# EUROPÄISCHE PATENTSCHRIFT

(11) **EP 2 156 813 B1**
(45) Veröffentlichungstag und Bekanntmachung des Hinweises auf die Patenterteilung: **16.01.2019**
(21) Anmeldenummer: 09166091.0
(22) Anmeldetag: 22.07.2009
(51) Int. Cl.: A61F 2/82, A61F 2/91, A61F 2/915

(54) **Stent und Verfahren zur Herstellung des Stents**
Stent and method for producing the stent
Stent et procédé de fabrication du stent

(30) Priorität: 19.08.2008 DE 102008038367
(43) Veröffentlichungstag der Anmeldung: 24.02.2010
(73) Patentinhaber: BIOTRONIK AG, 8180 Bülach (CH)
(72) Erfinder: Harder, Claus, 91080 Uttenreuth (DE)
(74) Vertreter: Keck, Hans-Georg

(56) Entgegenhaltungen:
- WO-A-98/55048
- WO-A-2006/086069
- US-A1- 2008 142 050
- US-B1- 6 685 737

## Beschreibung

Die vorliegende Erfindung betrifft einen Stent, der Struts umfasst. Des Weiteren betrifft die vorliegende Erfindung ein Verfahren zur Herstellung des erfindungsgemäßen Stents. Stents sind üblicherweise endovaskuläre Prothesen, die zur Therapie von Stenosen eingesetzt werden. Sie umfassen im Wesentlichen eine Tragstruktur, mittels derer die Wand eines Gefäßes, wie zum Beispiel einer Arterie, so abgestützt wird, dass ein ausreichender Durchfluss durch das Gefäß gewährleistet ist. Außerdem kann durch den Stent ein Aneurysma überbrückt werden. Bei der Implantation wird der Stent im komprimierten Zustand in das Gefäß eingeführt und anschließend an dem zu behandelnden Ort expandiert. Die Expansion des Stents erfolgt üblicherweise mittels eines Ballonkatheters, der zuvor in das Innere des Stents eingeführt wurde und der ebenfalls zur Positionierung des Stents im Gefäß dient. Durch die Expansion des Stents werden die Wände seiner Tragstruktur gegen die Gefäßwand gedrückt, sodass ein ausreichender Durchflussquerschnitt des Gefäßes realisiert wird. Damit nicht der Durchflussquerschnitt durch den Stent selbst zu stark verringert wird, weisen Stents im Allgemeinen sehr geringe Wandstärken im Bereich der Tragstruktur auf. Diese geringen Wandstärken müssen aber gewährleisten, dass trotz eines durch das Gefäß aufgebrachten radial auf den Stent wirkenden Druckes die expandierte Form des Stents erhalten bleibt. Neben der Radialfestigkeit besteht außerdem die Anforderung an den Stent, eine ausreichend geringe Biegesteifigkeit aufzuweisen, die es dem Stent ermöglicht, sich der teilweise gekrümmten Form und den Bewegungen des Gefäßabschnittes, in den er implantiert ist, möglichst gut anzupassen.
Die Tragstruktur des Stents ist dabei üblicherweise im Wesentlichen gitterförmig, wobei dieses Gitter unterschiedlichste Ausgestaltungen haben kann. Üblicherweise wird die Gitterstruktur durch ein auf eine Zylindermantelfläche eines Rohres ausgerichtetes Laserschneidverfahren erzeugt.

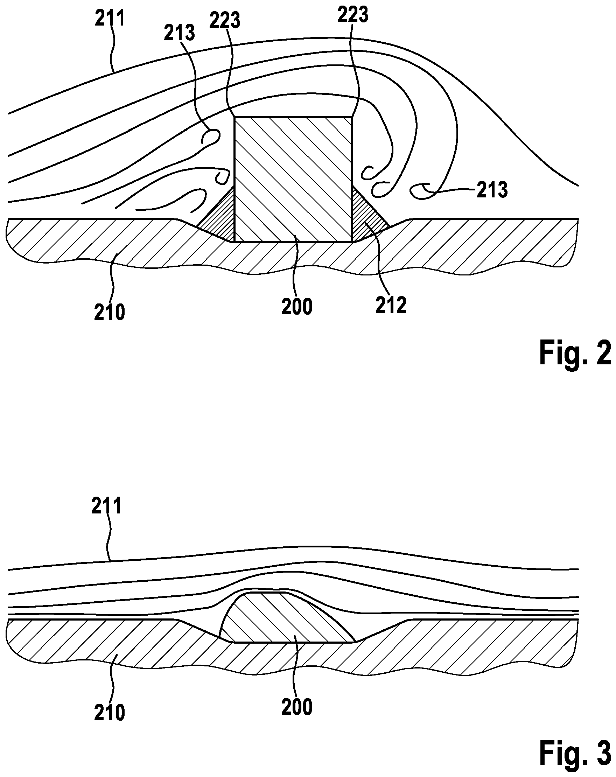

Die die Gitterstruktur ausbildenden Struts haben somit einen im Wesentlichen rechteckigen Querschnitt, wie in der den Stand der Technik darstellenden Figur 2 dargestellt. Nachteilig an der in Figur 2 gezeigten Ausführungsform des Struts 200 ist, dass der über den Strut 200 strömende Blutstrom 211 zu Verwirbelungen 213 neigt, sodass sich statt einer gewünschten laminaren Strömung eine turbulente Strömung im Bereich des Struts 200 einstellt. Die Verwirbelungen 213 führen zu unerwünschten Ablagerungen mit den möglichen Folgen der Neointimahyperlasie und Artherosklerose 212. Diese Ablagerungen können sich bis zu einer symptomatischen Restenose weiterentwickeln. Dieser Effekt tritt insbesondere bei den proximal liegenden Struts auf und nimmt in Richtung distal ab.

Es sind verschiedene Maßnahmen zur Bearbeitung der Struts nach dem Laserschneidverfahren bekannt. So werden zum Beispiel die Struts mit Elektropolitur behandelt. Die dadurch auftretende Kantenverrundung ist allerdings derart gering, dass sich keine nennenswerte Veränderung der Strömung von turbulent in laminar einstellt. Außerdem wird mit der Elektropolitur die gesamte Tragstruktur bearbeitet, sodass sich eine gezielte Bearbeitung des proximalen Endes des Stents nicht durchführen lässt. Auch erfolgt dabei der geringfügige Materialabtrag im Wesentlichen symmetrisch an den Struts, also an den muralen, den der Gefäßwand zugewandten, und an den luminalen, dem Gefäßlumen zugewandten Kanten, was der Bildung von Turbulenzen nicht entgegenwirkt.

Von der beschriebenen und in Figur 2 dargestellten Querschnittsform eines Struts abweichend ist eine ellipsenförmige Strut-Querschnittsfläche aus der EP 0 824 903 A1 bekannt. Aus deren Figur 2 ist eine Querschnittsform ersichtlich, bei der sowohl die luminale als auch die murale Oberfläche um die Längsachse des Struts herum gewölbt ist. Insbesondere die Wölbung der muralen Oberfläche hat dabei den Nachteil, dass ein derartig gewölbter Strut bei Dilatation tiefer in die Gefäßwand eindringt, wodurch diese stärker verletzt wird. Dies führt zu einer verstärkten Neointimaproliferation, was wiederum die Bildung von Ablagerungen begünstigt. Die luminale Oberfläche des Struts ist symmetrisch gewölbt. Es ist bekannt, dass symmetrische Wölbungen sich insofern ungünstig auf Flüssigkeitsströmungen auswirken, als dass sie keine laminare Strömung bewirken können. Das bedeutet, dass auch in dieser in der EP 0 824 903 A1 gezeigten ellipsenförmigen Ausführung Verwirbelungen des über die luminale Oberfläche hinwegströmenden Blutstroms auftreten, die ihrerseits ebenfalls zur Bildung von Ablagerungen führen. Ein weiterer Nachteil der in diesem Dokument genannten Strut-Querschnitte ist die Verringerung seines axialen Widerstandsmomentes, was sich insbesondere bei verstärkter Biegebeanspruchung nachteilig auf die Festigkeit des Stents auswirkt. Außerdem wird durch die verringerte Wandstärke der Tragstruktur die Radialfestigkeit des Stents herabgesetzt.
Insbesondere bei neuartigen, aus Magnesium-Legierungen bestehenden Stents werden aber aus Festigkeitsgründen relativ hohe Wandstärken gefordert, sodass die in der EP 0 824 903 A1 offenbarten Querschnittsformen von Struts für derartige Stents nicht anwendbar sind, ohne in unzulässiger Weise die Radialfestigkeit des Stents zu verringern. Um mit derartigen, aus dem zitierten Stand der Technik bekannten, Stegquerschnitten Stents aus Magnesium-Legierungen mit der geforderten Radialfestigkeit herzustellen, müssten die Struts im Querschnitt wesentlich größere Dimensionen aufweisen. Dies ist aber ungünstig, weil dadurch der Durchflussquerschnitt des Blutgefäßes verringert wird, was zu Turbulenzen und zu vermehrter Ablagerung führt.
In der WO 98/55048 A1, der WO 2006/086069 A2, der US 2008/0082162 A1 und der US 2008/0142050 A1 sind verschiedene Strut-Querschnitte offenbart, wobei diese allerdings auf ihrer muralen Oberfläche eine Beschichtung aufweisen. Wölbungen von luminalen Strut-Oberflächen sind symmetrisch ausgebildet, wodurch sich beim Einsatz im Blutstrom wieder die genannten unerwünschten Ablagerungen am Strut einstellen.
Der vorliegenden Erfindung liegt daher die Aufgabe zugrunde, einen Stent sowie ein Verfahren zur Herstellung des Stents zur Verfügung zu stellen, wobei der Stent derart ausgestaltet sein soll, dass bei seinem Einsatz eine Gefäßverengung minimiert wird und gleichzeitig eine ausreichende Biege- und Radialsteifigkeit des Stents gegeben ist.
Die Aufgabe wird durch den in Anspruch 1 genannten Stent gelöst. Weiterhin wird die Aufgabe durch das im Anspruch 6 genannte Verfahren zur Herstellung des Stents gelöst. Vorteilhafte Ausgestaltungen der jeweiligen Erfindungen schließen sich in den dazugehörigen Unteransprüchen an.
Es wird erfindungsgemäß ein Stent mit einer rohrähnlichen, einzelne Struts umfassenden Gitterstruktur zur Verfügung gestellt, wobei der Stent wenigstens einen Strut aufweist, von dem wenigstens ein Längenabschnitt mit wenigstens einer Richtungskomponente in radialer Umfangsrichtung des Stents verläuft, und wobei die radial nach außen gerichtete (murale) Längenabschnitts-Oberfläche des Struts lediglich um die Längsachse des Stents gekrümmt ist. Erfindungsgemäß weist der Stent lediglich in seinen Endbereichen Struts auf, die derart ausgestaltet sind, dass die zur Innenseite des Stents gerichtete (luminale) Oberfläche des Strut-Längenabschnittes durch verrundete luminale Kanten eine derartige Wölbung aufweist, dass der Strut-Querschnitt strömungstechnisch optimiert ist, wobei sich die Endbereiche, ausgehend von jeweils einem Stent-Ende (120, 130), über jeweils 20-30% der Länge des Stents (100) erstrecken. Die Erfindung betrifft somit die Ausgestaltung eines Struts oder auch nur eines Längenabschnittes eines Struts, der mit wenigstens einer Richtungskomponente in radialer Umfangsrichtung des Stents verläuft. Damit ist gemeint, dass der Strut beziehungsweise sein Längenabschnitt parallel zu einer am Umfang des Stents angelegten Tangente verläuft, beziehungsweise eine Komponente aufweist, die in diese Richtung weist. Erfindungsgemäß ausgeschlossen sind allerdings die Strut-Längenabschnitte, die lediglich parallel zur Längsachse des Stents verlaufen. Dies unterscheidet den erfindungsgemäßen Stent im Wesentlichen von den in der EP 0 824 903 A1 dargestellten Ausführungsformen. Derartige erfindungsgemäße Struts lassen sich in einfacher Weise mittels Laser aus einem Rohr herausschneiden. Unter dem Strut-Querschnitt wird der Querschnitt verstanden, der sich durch einen Schnitt in Längsrichtung des Stents durch den Längenabschnitt des Struts mit einer Richtungskomponente in radialer Umfangsrichtung ergibt, also mit der Richtung der Längsachse des Stents geführt ist. Erfindungsgemäß weist der Strut im Querschnitt in seiner Längsrichtung lediglich auf der luminalen Oberfläche eine Wölbung auf, die derart ausgestaltet ist, dass sie Turbulenzen von der durch den Stent strömenden Blutströmung und damit Ablagerungen verhindert. Auf der muralen Seite weist der Strut im in Längsrichtung des Stent verlaufenden Querschnitt keine Wölbung auf. Diese Ausgestaltung ist insbesondere deswegen von Vorteil, da sich dadurch die murale Auflagefläche des Struts an der Gefäßwand nicht verringert und somit der Druck vom Strut auf die Gefäßwand begrenzt wird, sodass die Gefahr von Gefäßbeschädigungen verringert wird.

Zur optimalen Vermeidung von Turbulenzen sollte der Stent erfindungsgemäß derart ausgestaltet sein, dass die luminale Oberfläche des Struts eine strömungstechnisch optimal gewölbte Oberfläche aufweist. Die optimale Wölbung ergibt sich für den Fachmann aus Versuchen oder aus herkömmlichen Strömungs-Simulationsmodellen und kann unter anderen von Durchflussquerschnitt des Gefäßes und der Strömungsgeschwindigkeit abhängig sein.

In bevorzugter Ausgestaltung des erfinderischen Stents ist vorgesehen, dass dessen zur Innenseite gerichtete Wölbung der Oberfläche des Struts asymmetrisch konvex ausgestaltet ist. Das heißt, dass die Wölbung der luminalen Oberfläche im Querschnitt betrachtet in jedem Punkt einen anderen Abstand zur muralen Oberfläche des Strut-Abschnittes aufweisen kann, wobei die konvexe Wölbung asymmetrisch ausgebildet ist. Mit anderen Worten weist die Wölbung der luminalen Oberfläche im Querschnitt betrachtet unterschiedliche konvexe Bereiche auf, die die Asymmetrie bedingen. Die Asymmetrie in der konvexen Ausgestaltung bewirkt eine stromlinienförmige Ausgestaltung des Strut-Querschnittes, wodurch der durch den Stent hindurchfließende Blutstrom im Bereich des Struts laminar strömt und somit Ablagerungen an den Struts vermieden werden. Gerade diese durch den Querschnitt hervorgerufene laminare Fließbewegung des Blutes hat den weiteren sehr wichtigen Effekt, dass die Endothelialisierung des Stents, also das "Einwachsen" mit Endothelzellen, begünstigt wird. Auch dies führt zur Verhinderung von Ablagerungen.

Es kann dabei vorgesehen sein, dass der erfindungsgemäße Stent im Bereich der Struts biodegradierbares Material umfasst. In weiterer Ausgestaltung kann vorgesehen sein, dass der Strut vollständig aus dem biodegradierbarem Material besteht. Als biodegradierbar im Sinne der Erfindung werden Materialien bezeichnet, bei denen in physiologischer Umgebung ein Abbau stattfindet, der letztendlich dazu führt, dass das gesamte Implantat oder der aus diesem Werkstoff gebildete Teil des Implantates seine mechanische Integrität verliert.

Vorzugsweise ist das biodegradierbare Material ein biodegradierbares Metall, bevorzugt eine biodegradierbare Legierung, ausgewählt aus der Gruppe Magnesium, Eisen und Wolfram; insbesondere ist das biodegradierbare metallische Material eine Magnesiumlegierung. Unter Legierung wird vorliegend ein metallisches Gefüge verstanden, deren Hauptkomponente Magnesium, Eisen oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Ist das Material eine Magnesiumlegierung, so enthält diese vorzugsweise Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet.

Besonders bevorzugt wird eine Magnesiumlegierung der Zusammensetzung Seltenerdmetalle 5,2 - 9,9 Gew.%, davon Yttrium 0,0 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Degradationsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden. Die Legierungen der Elemente Magnesium, Eisen oder Wolfram sind so in ihrer Zusammensetzung zu wählen, dass sie biodegradierbar sind.

Alternativ kann anstatt eines auf Metall basierenden Materials ein biodegradierbares Polymer zur Anwendung kommen. Bevorzugte Polymere für die Polymermatrix des erfindungsgemäßen Implantats werden ausgewählt aus der Gruppe Polydioxanon, Polyglycolid Polycaprolacton, Polylactide (Poly-L-Lactid, Poly-D,L-Lactid, und Copolymere sowie Blends wie Poly(L-Lactid-co-glycolid), Poly(D,L-lactid-co-glycolid), Poly(L-Lactid-co-D,L-Lactid), Poly(I-Lactid-co-trimethylen carbonat, Triblockcopolymere), Polysaccharide (Chitosan, Levan, Hyaluronsäure, Heparin, Dextran, Cellulose etc.), Polyhydroxyvalerat, Ethylvinylacetat, Polyethylenoxid, Polyphosphorylcholin, Fibrin, Albumin.

Alternativ kann auch vorgesehen sein, dass der Strut aus einem permanenten Material besteht, welches sich nicht auflöst. Der Grundkörper dieser Permanentstents besteht vorzugsweise aus einem metallischen Material aus einem oder mehreren Metallen aus der Gruppe Eisen, Nickel, Wolfram, Titan, Zirkonium, Niob, Tantal, Zink oder Silizium und ggf. einer zweiten Komponente aus einem oder mehreren Metallen aus der Gruppe Lithium, Natrium, Kalium, Kalzium, Mangan Eisen oder Wolfram, vorzugsweise aus einer Zink-Kalziumlegierung. In einem weiteren Ausführungsbeispiel besteht der Grundkörper aus einem Formgedächtnis-Material aus einem oder mehreren Materialien aus der Gruppe bestehend aus Nickel-Titan-Legierungen und Kupfer-Zink-AluminiumLegierungen, vorzugsweise aber aus Nitinol. In einem weiteren bevorzugten Ausführungsbeispiel besteht der Grundkörper des Stents aus Edelstahl, vorzugsweise aus einem Cr-Ni-Fe-Stahl - hier bevorzugt die Legierung 316L - oder einem Co-Cr-Stahl.

Ferner kann der Grundkörper des Stents mindestens teilweise aus Kunststoff und/oder einer Keramik bestehen.

In einer Ausgestaltung der Erfindung ist vorgesehen, dass sämtliche Strut-Abschnitte des Stents, die mit einer Richtungskomponente senkrecht zur Längsrichtung des Stents verlaufen, gemäß der vorliegenden Erfindung ausgestaltet sind. In der erfindungsgemäßen Ausgestaltung weist der Stent Struts, die gemäß der Erfindung ausgestaltet sind, lediglich in seinen Endbereichen auf, wobei sich die Endbereiche, ausgehend von jeweils einem Stent-Ende, über jeweils 20 bis 30 Prozent der Länge des Stents erstrecken. Das heißt, dass nur an den Stent-Endbereichen die Struts strömungstechnisch optimiert ausgebildet sind. Der mittlere Bereich des Stents wird somit - abgesehen von der herkömmlichen Elektropolitur zur Glättung der Strutoberflächen und zur Vermeidung scharfer Strutkanten, die auch weiterhin angewandt wird - keiner Strut-Oberflächenbearbeitung unterzogen, die die Form der Strutquerschnitte signifikant verändert,. Dies kann für einfache Stents ausreichend sein, da erfahrungsgemäß die Ablagerungen nur in den Endbereichen des Stents (meist am proximalen Stent-Ende) auftreten und nicht in der Stent-Mitte. Diese Ausgestaltung hat den Vorteil, dass die Wandstärke des Stents, also die Materialdicke der Struts im Mittenbereich des Stents, dicker ausgeführt ist als zumindest am proximalen Stent-Ende. Es ist dabei auch möglich, den Stent derart auszuführen, dass über die Länge des Stents eine unterschiedlich starke Oberflächenbearbeitung vorgenommen wird, sodass die Querschnittsfläche des Struts umso größer ist, je zentraler er im Stent angeordnet ist. Das heißt, dass der Materialabtrag zur Realisierung der strömungstechnischen Optimierung des Strut-Querschnittes umso größer ist, je weiter ein Strut am Ende des Stents angeordnet ist,. Dies wiederum hat den Vorteil, dass bei einem Angriff eines Biegemomentes am Stent-Ende insbesondere in dem Bereich des Stents die Wandstärke am dicksten ausgeführt ist, in dem die größte Biegespannung vorliegt. Die Stent-Enden bleiben dabei flexibel und können sich somit leichter Gewebe-Bewegungen anpassen. Ein weiterer Vorteil ist, dass der Übergang zwischen der Region, in der die Gefäßwand von einem Stent dieser Ausführung gestützt wird, und der direkt an die Stent-Enden angrenzenden Gefäßwand "weich" ist. Das bedeutet, dass die Elastizität des Gefäßes ohne Abstufung zunimmt bzw. abnimmt. Mit anderen Worten wird ein abrupter Abfall der Elastizität zwischen ungestützter Gefäßwand und Gefäßwand mit Stent vermieden. Dadurch wird die mechanische Reizung an den Stentenden minimiert, was wiederum das Risiko fokaler Stenosen an den Stent-Enden verringert.
In einer bevorzugten Ausführungsform ist vorgesehen, dass der erfindungsgemäße Stent spiegelsymmetrisch aufgebaut ist. Das bedeutet, dass zum Beispiel konvexe Asymmetrien an den Oberflächenwölbungen der luminalen Strut-Oberflächen derart angeordnet und ausgeprägt sind, dass sie auf jeder Hälfte des Stents eine andere, jeweils entgegengesetzte Ausrichtung aufweisen. Die Spiegelebene verläuft dabei senkrecht zur Stentlängsachse genau in der longitudinalen Mitte des Stents. Durch diese Ausgestaltung ist zwar ein Ende des Stents aufgrund seiner Asymmetrie und Lage im Gefäß bei dessen Einsatz im Blutstrom strömungstechnisch nicht optimal ausgestaltet, dieser Nachteil ist aber deswegen akzeptabel, weil dieser Stentbereich das distale Ende des Stents bildet, an dem nur minimale Ablagerungen aufgrund von sehr geringen Turbulenzen auftreten. Auf jeden Fall ist aber das proximale Ende des Stents strömungstechnisch optimiert, sodass an diesem Ende Ablagerungen erfindungsgemäß vermieden werden. Diese Ausgestaltung weist den Vorteil auf, dass der Einsatz des Stents ausrichtungsunabhängig ist, da jedes der Enden des Stents strömungstechnisch optimiert ist und somit unabhängig von der Ausrichtung des Stents bei dessen Einsatz das proximale Ende des Stents bilden kann.

Es kann auch vorgesehen sein, dass die Asymmetrien der Wölbungen an den Struts über die gesamte Länge des Stents einseitig ausgerichtet sind. Somit existiert keine Spiegelsymmetrie des Stents mehr, dafür ergibt sich aber der Vorteil, dass beide Enden des Stents strömungstechnisch optimiert ausgestaltet sind und somit auch die eventuell minimal auftretenden Ablagerungen am distalen Ende vermieden werden können. Bei dieser Ausgestaltung muss allerdings bei der Implantation beachtet werden, dass das proximale Ende des Stents das hinsichtlich der Durchflussrichtung optimierte Ende ist.
Erfindungsgemäß ausgestaltete Strut-Querschnitte können eine Dicke von 60 bis 185 µm, vorzugsweise von 60 bis 90 µm aufweisen. Das heißt, dass die Wanddicke der Tragstruktur des erfindungsgemäßen Stents ebenfalls in diesen angegebenen Mikrometer-Bereichen liegt.

Ein weiterer Aspekt ist die Verwendung des erfindungsgemäßen Stents als Implantat in einem Gefäß zur Verhinderung der Gefäßverengung. Der erfindungsgemäße Stent bezieht sich somit auf eine Ausführungsform, die derart hergestellt ist, dass sie die strömungstechnisch optimierte gewölbte Oberfläche auf der luminalen Seite des Struts bereits bei der Implantation aufweist. Dies unterscheidet den erfindungsgemäßen Stent von Ausführungsformen, bei denen sich erst nach relativ langem Einsatz des Stents im Gefäß aufgrund von Material-Erosion des Struts ein an den Blutfluss zumindest teilweise angepasster Strut-Querschnitt ergibt. Mittels des erfindungsgemäßen Stent lassen sich somit unmittelbar nach der Implantation die Ablagerungen verhindern.

Erfindungsgemäß wird außerdem ein Verfahren zur Herstellung des erfindungsgemäßen Stents zur Verfügung gestellt, bei dem die Gitterstruktur des Stents derart hergestellt wird, dass zumindest ein mit wenigstens einer Richtungskomponente in radialer Umfangsrichtung des Stents verlaufender Längenabschnitt eines Struts einen im Wesentlichen eckigen Querschnitt aufweist, wobei eine Verrundung der luminalen und mit wenigstens einer Richtungskomponente in radialer Umfangsrichtung des Stents verlaufenden Kanten des Längenabschnitts des Struts durch wenigstens einen auf die Kanten gerichteten Partikelstrahl erfolgt, der aus einer Düse am Ende einer Transportleitung austritt, der in den Stent eingeführt ist.

In üblicher Weise wird die Gitterstruktur durch Laserschneiden eines Rohres erzeugt. Der Partikelstrahl kann dabei als Partikel feinkörnigen Sand oder Kügelchen aus einem festen Material umfassen. Aufgrund einer Streuung des Partikelstrahls trifft dieser auch auf Kanten, die nicht direkt im Projektionsbereich einer Düse, aus der der Partikelstrahl ausströmt, angeordnet sind. Durch eine leichte Taumelbewegung oder kreisende Bewegung des Partikelstrahls kann sichergestellt werden, dass die Partikel in ausreichender Weise alle vor der Düse in Reichweite des Strahls befindlichen Strut-Kanten treffen. Es lässt sich somit eine derartige Verrundung der luminalen Kanten der Struts herstellen, dass diese den erfindungsgemäßen, strömungstechnisch optimierten Querschnitt aufweisen.

Es ist dabei vorteilhafterweise vorgesehen, dass die beiden luminalen Kanten des Strut-Längenabschnittes mit jeweils einem Partikelstrahl bearbeitet werden, wobei die Richtungen der beiden Partikelstrahle entgegengesetzt sind.

Das Verfahren ist dann vorteilhaft ausgebildet, wenn der Partikelstrahl aus einer in den Stent eingeführten Düse auf die in Richtung der Düse weisende Kante des Struts ausgerichtet wird und die Düse zur sequentiellen Bestrahlung mehrerer in Längsrichtung des Stents nebeneinander angeordneter Struts mit bestimmter Geschwindigkeit aus dem Stent herausgezogen wird. Dabei erfolgt eine Streuung des Partikelstrahls, sodass alle in einem Schnitt quer zur Längsrichtung des Stents liegenden Strut-Kanten gleichzeitig bestrahlt werden.

Vorteilhafterweise erfolgt die Bestrahlung der Kanten dabei beidseitig, wobei die Düse wenigstens einmal aus dem Stent in Richtung des ersten Stent-Endes und wenigstens einmal aus dem Stent in Richtung des zweiten Stent-Endes gezogen wird. Dabei muss die Düse nicht vollständig in den Stent eingeführt worden sein, sondern es reicht zur Bearbeitung der Stent-Endbereiche aus, wenn die Düse nur in diesen Endbereich eingeführt wird und bei Aufbringung des Partikelstrahls wieder aus dem Stent herausgezogen wird. Alternativ wird die Düse vollständig in den Stent eingeschoben und wird von dem dem Einschubende gegenüberliegenden Ende bei Aufbringung des Partikelstrahls lediglich durch diesen gegenüberliegenden Endbereich gezogen, um somit diesen Endbereich von der anderen Seite zu bestrahlen.
Zur Verrundung der beiden luminalen Kanten des Längenabschnitts des Struts sollten beide Verfahrensvarianten angewendet werden. Dabei sind die beiden Varianten mit unterschiedlicher Partikeldichte, unterschiedlichem Volumenstrom, unterschiedlicher Geschwindigkeit des Herausziehens der Düse und/ oder unterschiedlicher Anzahl der Strahlvorgänge durchführbar, um die asymmetrisch konvexe Wölbung der luminalen Oberflächen der Struts zu realisieren.

Zur abschließenden Bearbeitung des Stents ist vorgesehen, dass nach dem Partikelstrahlen der Stent mittels Elektropolitur bearbeitet wird. Die Elektropolitur dient dabei der Verrundung der Kanten, wobei neben den luminalen Kanten auch die muralen Kanten des Struts etwas verrundet werden, um ein Einschneiden in der Gefäßwandung zu verhindern. Trotz der Verrundung der muralen Kanten wird die murale Oberfläche des Struts allerdings nicht derart umfassend geändert, dass sich eine ingesamte Wölbung der Oberfläche einstellt.
Die Elektropolitur dient außerdem der abschließenden Oberflächen-Glättung der gesamten Tragstruktur des Stents.

Vorteilhafterweise ist vorgesehen, dass die Geschwindigkeit der Düse beim Herausziehen variiert wird. Das heißt, dass zum Beispiel beim Herausziehen der Düse aus einem Endbereich des Stents die Düse zuerst schneller und dann langsamer herausgezogen wird, sodass an der zur Mitte des Stents gewandten Seite des Endbereiches weniger Materialabtrag realisiert wird als am Stent-Ende.
Eine Vorrichtung zur Durchführung des erfindungsgemäßen Verfahrens wird zur Verfügung gestellt, wobei diese Vorrichtung ein Reservoir zur Bereitstellung von Strahlpartikeln, eine Einrichtung zur Erzeugung von Überdruck, eine Leitung zum Transport der Strahlpartikel und eine mit der Transportleitung gekoppelte Düse zur Ausbringung der Strahlpartikel in Strahlform umfasst, wobei zumindest die Düse derartige geometrische Maße aufweist, dass sie in einen Stent einführbar ist. Das Reservoir kann zum Beispiel ein Behälter sein, in dessen Innerem ein Überdruck herrscht. Zur Erzeugung des Überdrucks kann eine herkömmliche Pumpe verwendet werden. Die Transportleitung kann ein Schlauch oder ein Rohr sein, an welchem eine Düse angeschlossen ist. Dabei kann die Transportleitung auch derart ausgestaltet sein, dass die Düse durch ein offenes Ende der Transportleitung hergestellt ist, wenn dieses Ende geeignet ist, in einen Stent eingeführt zu werden und einen Partikelstrahl zu erzeugen. In diesem Fall ist die Düse ein integraler Bestandteil der Transportleitung.
Der erfindungsgemäße Stent lässt sich somit mittels dem erfindungsgemäßen Verfahren an den luminalen Kanten derart verrunden, dass die Blutströmung an den erfindungsgemäß ausgestalteten Struts laminar erfolgt. Die muralen Kanten werden dabei nicht über das mit normaler Elektropolitur Erreichbare hinaus verrundet, um die murale Auflagefläche möglichst groß zu halten und somit den Druck auf die Gefäßwand zur Verhinderung von Verletzungen gering zu halten. Die asymmetrische Querschnittsform des Struts ist strömungstechnisch optimiert, wobei sie entsprechend strömungstechnischer Simulationsmodelle ausgestaltet sein kann. Es wird zum Beispiel eine asymmetrische Verrundung der luminalen Kanten an den Stent-Endbereichen vorgenommen, wobei insbesondere das proximale Ende des Stents strömungstechnisch optimiert ist. Dadurch wird die Bildung von Ablagerungen an den Struts vermieden und somit die Gefahr von unerwünschten Neointimahyperlasien und arteriosklerotischen Erscheinungen verringert. Dadurch, dass bevorzugt der Materialabtrag an den Stent-Endbereichen erfolgt, ist lediglich in diesen Stent-Endbereichen die Wandstärke der Tragstruktur verringert. Das bedeutet, dass im mittleren Bereich des Stents dieser eine Wandstärke der Tragstruktur aufweist, die der Wandstärke des Rohres entspricht, aus dem die Tragstruktur herausgeschnitten ist. Daraus ergibt sich eine verringerte Biegesteifigkeit an den Stent-Enden und eine im Vergleich dazu erhöhte Biegefestigkeit in der Stent-Mitte. Diese Eigenschaften sind insbesondere dann vorteilhaft, wenn der Stent im Gebrauch Bewegungen des Gefäßes, in welches er eingesetzt ist, folgen muss. Das heißt, dass der erfindungsgemäß ausgestaltete Stent leichter als herkömmliche Stents die Bewegung des Gefäßes mitmachen kann, wodurch Absätze beziehungsweise Spalten zwischen dem Stent-Ende und der daran anliegenden Gefäßwand vermieden werden, wodurch wiederum die Gefahr der Bildung von Ablagerungen in diesem Bereich verringert wird.

Die Erfindung wird anhand der beiliegenden Zeichnungen beschrieben. Es zeigt dabei
- Figur 1:: einen Ausschnitt aus einer möglichen Tragstruktur eines erfindungsgemäßen Stents,
- Figur 2:: den Querschnitt eines herkömmlichen, quer zur Längsrichtung des Stents verlaufenden Struts im Einsatz,
- Figur 3:: den Querschnitt eines erfindungsgemäß ausgestalteten, quer zur Längsrichtung des Stents verlaufenden Struts,
- Figur 4:: einen schematisch angedeuteten Stent mit darin eingeführter Düse und Partikelstrahl,
- Figur 5:: schematische Darstellung einzelner Bearbeitungsstufen der Struts.

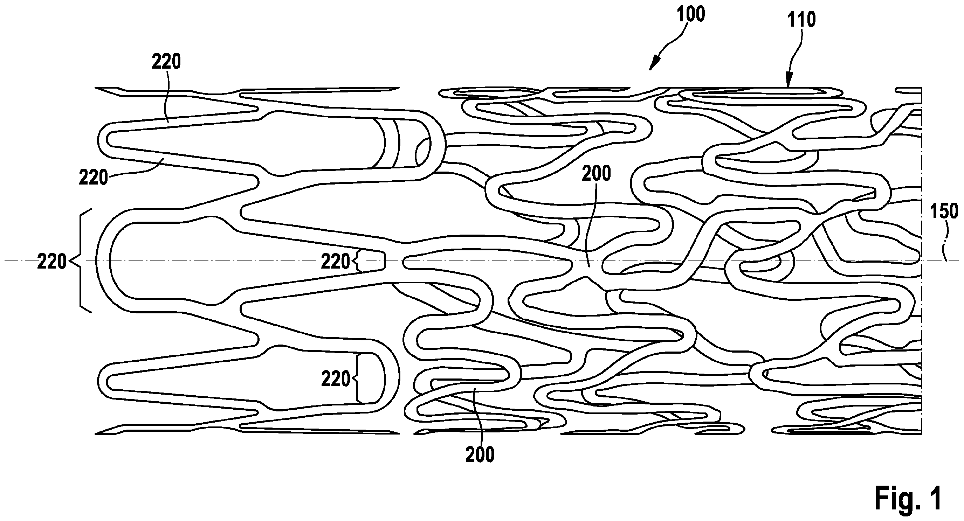

In den Figuren 1 und 2 sind Struts 200 dargestellt, wie sie herkömmlich in der Tragstruktur beziehungsweise Gitterstruktur 110 eines Stents 100 verwendet werden. Die Erfindung ist dabei nicht auf das Design der in Figur 1 dargestellten Gitterstruktur 110 eingeschränkt, sondern die Erfindung kann sämtliche Stentstrukturen umfassen, die Struts aufweisen, und deren Längenabschnitte zumindest eine Richtungskomponente aufweisen, die in radialer Umfangsrichtung des Stents verläuft. Damit sind diejenigen Längenabschnitte 220 gemeint, die, wie in Figur 1 dargestellt, nicht parallel zur Längsachse 150 des Stents 100 verlaufen. Somit betrifft dies Längenabschnitte 220, die offensichtlich in radialer Umfangsrichtung verlaufen, aber auch die Längenabschnitte die schräg zur Längsachse verlaufen, da auch diese Längenabschnitte eine Richtungskomponente aufweisen, die senkrecht zur Längsachse führt.

Es ist aus Figur 2 ersichtlich, dass die zur Außenseite des Stents gerichteten Flächen der Struts, also die muralen Oberflächen, die an der Gefäßwand 210 anliegen, im gezeigten Querschnitt keine Krümmung aufweisen. Das heißt, dass diese Oberflächen lediglich in einer Richtung gekrümmt sind, nämlich um die Längsachse 150. Eine Krümmung der muralen Strut-Oberflächen um Achsen, die senkrecht zur Längsachse 150 stehen, ist beim erfindungsgemäßen Stent nicht realisiert.

Die Erfindung wird insbesondere durch die Gegenüberstellung der erfindungsgemäßen Querschnittsform des Struts gemäß Figur 3 und der herkömmlichen Strut-Querschnittsform in Figur 2 deutlich. Durch die luminalen Kanten 223 an einem herkömmlichen Strut 200, wie in Figur 2 gezeigt, entsteht eine Verwirbelung 213 des Blutstroms 211, die zur Bildung von Ablagerungen 212 zwischen der Gefäßwand 210 und dem Strut 200 führt. Diese Ablagerungen können sich arteriosklerotisch auswirken und sind zu verringern beziehungsweise zu verhindern.
Durch die erfindungsgemäße Ausgestaltung des Struts 200, wie in Figur 3 gezeigt, wird der Querschnitt des Struts 200 derart optimiert, dass sich ein laminarer Blutstrom 211 ausbildet. Ablagerungen werden dadurch vermieden.

Es ist aus Figur 3 ersichtlich, dass der Querschnitt des Struts 200 asymmetrisch konvex ausgeführt ist. Insbesondere durch die Asymmetrie wird die Ausbildung der laminaren Strömung begünstigt.

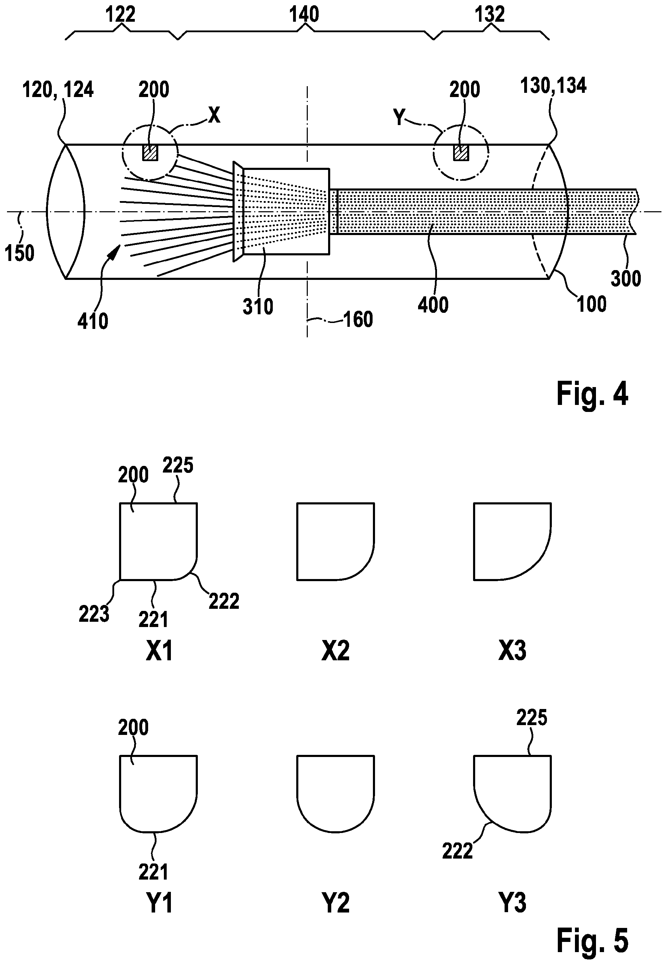

In Figur 4 ist dargestellt, auf welche Weise die strömungstechnisch optimierte Verrundung an den Struts herstellbar ist. Es ist ein schematisch dargestellter Stent 100 ersichtlich, der durch eine Mehrzahl von Struts 200, ähnlich oder identisch dem in Figur 1 dargestellten Design, ausgebildet ist. Aus Gründen der Übersichtlichkeit ist in Figur 4 auf jeder Seite des Stents nur ein Strut 200 im Querschnitt dargestellt, der in vergrößerten Ansichten ebenfalls in Figur 5 gezeigt ist.

Zur Durchführung des erfindungsgemäßen Verfahrens ist in den Stent 100 eine Transportleitung 300 eingeführt, an deren Ende eine Düse 310 angeordnet ist. Die Transportleitung 300 transportiert Partikel 400 zur Düse 310, aus der die Partikel 400 als Partikelstrahl 410 austreten. Der Partikelstrahl 410 weist eine gewisse Streuung auf, sodass die aus der Düse 310 austretenden Partikel 400 seitlich auf die Struts 200 und ebenfalls auf die luminale Längenabschnitt-Oberfläche 221 des Struts 200 auftreffen. Durch die aufprallenden Partikel 400 wird erreicht, dass die luminalen Kanten 223 verrundet werden. Die Düse 310 wird in Richtung des zweiten Stent-Endes 130 aus dem Stent 100 herausgezogen. Alternativ kann die Düse auch in Richtung des ersten Stent-Endes 120 durch den Stent 100 hindurchgeschoben werden, wobei allerdings zu beachten ist, dass keine Behinderung der Verschiebung der Düse 310 durch beim Strahlvorgang zunächst im Stent 100 verbleibende Reste von Partikeln 400 erfolgt.

Durch mehrmaliges beziehungsweise länger anhaltendes Bestrahlen des Struts 200 lässt sich wie in Figur 5 in den Einzeldarstellungen der Vergrößerung des Bereichs X aus Figur 4 erkennen, dass eine luminale Kante 223 des Struts 200 verrundet wird. Dabei stellt sich eine Wölbung 222 heraus, deren Radius umso größer ist, je länger oder öfter der Strahl auf den Strut 200 gerichtet ist.
In den Einzeldarstellungen Y1 bis Y3 in Figur 5 ist der Strut 200 in vergrößerter Darstellung des mit Y gekennzeichneten Bereichs in Figur 4 gezeigt. Es ist ersichtlich, dass bei dem hier dargestellten Strut 200 die Verrundung bereits an beiden luminalen Kanten 223 stattgefunden hat, sodass sich an der luminalen Längenabschnitts-Oberfläche 221 eine Wölbung 222 ausgebildet hat, die, wie insbesondere aus der Darstellung Y3 ersichtlich, asymmetrisch konvex ausgebildet ist. Zur Erreichung dieser mit Y3 in Figur 5 dargestellten Querschnittsform des Struts wird wie in Figur 4 dargestellt die Düse 310 zunächst vom ersten Stent-Ende 120 in Richtung des zweiten Stent-Endes 130 und weiterhin aus dem Stent 100 heraus gezogen. Dieser Vorgang lässt sich wiederholt durchführen. Es ergeben sich damit die in den Darstellungen X1 bis X3 angegebenen Querschnittsformen.
Zur Verrundung der noch bestehenden luminalen Kante 223 wird die Düse 310 in ähnlicher Weise wie beschrieben vom zweiten Stent-Ende 130 in Richtung des ersten Stent-Endes 120 gezogen und aus dem Stent herausgezogen. Dadurch ergibt sich, wie in Y3 zu Figur 5 dargestellter Querschnittsform gezeigt, bei längerer oder mehrmaliger Bestrahlung ein weiterer Materialabtrag, der zur asymmetrisch konvexen Form des Querschnittes führt.

Die Erfindung ist allerdings nicht auf diese Verfahrensweise begrenzt, sondern es kann auch vorgesehen sein, dass die Düse 310 lediglich von der Stent-Mitte 140 jeweils zum ersten Stent-Ende 120 und zum zweiten Stent-Ende 130 gezogen wird, um somit die entsprechenden luminalen Kanten 223 zu verrunden. Es ist dabei bevorzugt vorgesehen, dass lediglich die Struts 200 verrundet werden, die in den beiden Endbereichen 122 und 132 angeordnet sind, sodass die sich in der Stent-Mitte 140 befindlichen Struts 200 nicht durch den Partikelstrahl 410 verrundet werden.

Es kann außerdem vorgesehen sein, dass der Stent 100 spiegelsymmetrisch ausgebildet ist, sodass seine beiden Hälften entlang der Spiegelsymmetrie-Achse 160 symmetrisch ausgebildet sind. In diesem Fall sind die in den Bereichen X und Y in Figur 4 dargestellten Struts 200 derart ausgeführt, dass ihre konvexen Wölbungen unterschiedliche und entgegengesetzte Ausrichtungen haben. Eine solche Ausgestaltung hat den Vorteil, dass der erfindungsgemäße Stent orientierungsunabhängig in das Gefäß eingesetzt werden kann, da sein proximales Ende 134 auf jeden Fall strömungstechnisch optimiert ausgestaltet ist. Die in diesem Fall strömungstechnisch etwas ungünstigere Ausgestaltung des distalen Endes 124 wirkt sich dabei nicht störend aus, da am distalen Ende keine oder nur minimale Ablagerungen zu erwarten sind.

Alternativ kann der Stent auch derart ausgeführt sein, dass alle asymmetrisch konvexen Wölbungen dieselbe Ausrichtung aufweisen. Das heißt, dass alle Strut-Querschnitte zum Beispiel die Form wie in Y3 der Figur 5 dargestellt aufweisen können. Diese Ausgestaltung hat den Vorteil der strömungstechnischen Ausgestaltung aller Struts 200, wobei allerdings bei der Implantation des Stents zu beachten ist, dass der Stent 100 derart in das Gefäß eingeführt wird, dass das hinsichtlich der Durchflussrichtung optimierte Ende das proximale Ende 134 des Stents 100 ist.

Aus den Darstellungen aus Figur 5 ist ersichtlich, dass die murale Längenabschnitts-Oberfläche 225 durch den Partikelstrahl nicht verrundet wird.

**Bezugszeichenliste**

| | |
|---|---|
| Stent | 100 |
| Gitterstruktur | 110 |
| Erstes Stent-Ende | 120 |
| Erster Endbereich | 122 |
| Distales Ende | 124 |
| Zweites Stent-Ende | 130 |
| Zweiter Endbereich | 132 |
| Proximales Ende | 134 |
| Stent-Mitte | 140 |
| Längsachse | 150 |
| Spiegelsymmetrie-Achse | 160 |
| | |
| Strut | 200 |
| Gefäßwand | 210 |
| Blutstrom | 211 |
| Ablagerung | 212 |
| Verwirbelung | 213 |
| | |
| Längenabschnitt | 220 |
| luminale Längenabschnitts-Oberfläche | 221 |
| Wölbung | 222 |
| luminale Kante | 223 |
| | |
| murale Längenabschnitts-Oberfläche | 225 |
| | |
| Transportleitung | 300 |
| Düse | 310 |
| | |
| Partikel | 400 |
| Partikelstrahl | 410 |

## Patentansprüche

1. Stent (100) mit einer rohrähnlichen, einzelne Struts umfassenden Gitterstruktur (110), wobei der Stent wenigstens einen Strut (200) aufweist, von dem wenigstens ein Längenabschnitt (220) mit wenigstens einer Richtungskomponente in radialer Umfangsrichtung des Stents verläuft und wobei die zur Außenseite des Stents gerichtete Längenabschnitts-Oberfläche lediglich um die Längsachse (150) des Stents gekrümmt ist,
**dadurch gekennzeichnet, dass**
der Stent (100) lediglich in seinen Endbereichen (122, 132) Struts (200) aufweist, die derart ausgestaltet sind, dass die zur Innenseite des Stents gerichtete Oberfläche (221) des Strut-Längenabschnittes (220) durch verrundete luminale Kanten eine derartige Wölbung (222) aufweist, dass der Strutquerschnitt strömungstechnisch optimiert ist, um die Turbulenzen von der durch den Stent strömenden Blutströmung zu verhindern, wobei sich die Endbereiche, ausgehend von jeweils einem Stent-Ende (120, 130), über jeweils 20-30% der Länge des Stents (100) erstrecken.

2. Stent nach Anspruch 1, **dadurch gekennzeichnet, dass** die zur Innenseite des Stents (100) gerichtete Wölbung (222) der Oberfläche des Struts (200) asymmetrisch konvex ausgestaltet ist.

3. Stent nach einem der vorhergehenden Ansprüche, **dadurch gekennzeichnet, dass** der Strut (200) biodegradierbares Material umfasst.

4. Stent nach einem der vorhergehenden Ansprüche, **dadurch gekennzeichnet, dass** sämtliche Strutabschnitte des Stents (100), die mit wenigstens einer Richtungskomponente in radialer Umfangsrichtung des Stents verlaufen, gemäß einem der Ansprüche 1 bis 3 ausgestaltet sind.

5. Stent nach einem der vorhergehenden Ansprüche, **dadurch gekennzeichnet, dass** er spiegelsymmetrisch aufgebaut ist.

6. Verfahren zur Herstellung eines Stents (100) gemäß einem der Ansprüche 1 bis 5, bei dem die Gitterstruktur (110) des Stents derart hergestellt wird, dass zumindest ein mit wenigstens einer Richtungskomponente in radialer Umfangsrichtung des Stents verlaufender Längenabschnitt (220) eines Struts (200) einen im wesentlichen eckigen Querschnitt aufweist, wobei eine Verrundung der luminalen und mit wenigstens einer Richtungskomponente senkrecht zur Längsrichtung des Stents verlaufenden Kanten (223) des Längenabschnittes (220) des Struts (200) durch wenigstens einen auf die Kanten (223) gerichteten Partikelstrahl erfolgt, **dadurch gekennzeichnet, dass** der Partikelstrahl aus einer Düse (310) am Ende einer Transportleitung (300) austritt, die in den Stent (100) eingeführt ist.

7. Verfahren zur Herstellung eines Stents nach Anspruch 6, **dadurch gekennzeichnet, dass** die beiden luninalen Kanten (223) des Strut-Längenabschnittes (220) mit jeweils wenigstens einem Partikelstrahl (410) bearbeitet werden, wobei die Richtungen der beiden Partikelstrahle (410) entgegengesetzt sind.

8. Verfahren zur Herstellung eines Stents nach Anspruch 7 , **dadurch gekennzeichnet, dass** der Partikelstrahl (410) aus der in den Stent (100) eingeführten Düse (310) auf die in Richtung der Düse (310) weisenden Kanten (223) der Struts (200) ausgerichtet wird und die Düse (310) zur sequentiellen Bestrahlung mehrer in Längsrichtung des Stents nebeneinander angeordneten Struts (200) mit bestimmter Geschwindigkeit aus dem Stent (100) herausgezogen wird.

9. Verfahren zur Herstellung eines Stents nach einem der Ansprüche 7 oder 8, **dadurch gekennzeichnet, dass** die Bestrahlung der Kanten (223) beidseitig erfolgt, wobei die Düse (310) wenigstens einmal aus dem Stent (100) in Richtung des ersten Stent-Endes (120) und wenigstens einmal aus dem Stent (100) in Richtung des zweiten Stent-Endes (130) gezogen wird.

10. Verfahren zur Herstellung eines Stents nach einem der Ansprüche 7 bis 9, **dadurch gekennzeichnet, dass** nach dem Partikelstrahlen der Stent (100) mittels Elektropolitur bearbeitet wird.

11. Verfahren zur Herstellung eines Stents nach einem der Ansprüche 8 bis 10, **dadurch gekennzeichnet, dass** die Geschwindigkeit der Düse (310) beim Herausziehen variiert.

## Claims

1. A stent (100), comprising a tubular lattice structure (110) comprising individual struts, wherein the stent has at least one strut (200), of which at least one longitudinal portion (220) runs with at least one directional component in the radial circumferential direction of the stent, and wherein the surface of the longitudinal portion facing the outside of the stent is curved only around the longitudinal axis (150) of the stent,
**characterised in that**
the stent (100) merely in its end regions (122, 132) has struts (200) which are formed in such a way that the surface (221) of the strut longitudinal portion (220) facing towards the inside of the stent has a curvature (222), caused by rounded luminal edges, such that the strut cross section is fluidically optimised in order to prevent turbulences of the blood flow flowing through the stent, wherein the end regions, starting from each stent end (120, 130), extend in each case over 20-30% of the length of the stent (100).

2. The stent according to claim 1, **characterised in that** the curvature (222) of the surface of the strut (200), which curvature is directed towards the inside of the stent (100), is of asymmetrically convex shape.

3. The stent according to either one of the preceding claims, **characterised in that** the strut (200) comprises a biodegradable material.

4. The stent according to any one of the preceding claims, **characterised in that** all of the strut sections of the stent (100) which run with at least one directional component in the radial circumferential direction of the stent are formed in accordance with any one of claims 1 to 3.

5. The stent according to any one of the preceding claims, **characterised in that** the stent is constructed in mirror-symmetrical fashion.

6. A method for producing a stent (100) according to any one of claims 1 to 5, in which the lattice structure (110) of the stent is produced in such a way that at least one longitudinal portion (220) of a strut (200) running with at least one directional component in the radial circumferential direction of the stent has a substantially angular cross section, wherein there is a rounding of the luminal edges (223) of the longitudinal portion (220) of the strut (200), and of said edges (223) running with at least one directional component perpendicular to the longitudinal direction of the stent, by means of at least one particle beam directed towards the edges (223), **characterised in that** the particle beam exits from a nozzle (310) at the end of a transport conduit (300) which is inserted into the stent (100).

7. The method for producing a stent according to claim 6, **characterised in that** the two luminal edges (223) of the longitudinal portion (220) of the strut are each treated by at least one particle beam (410), wherein the directions of the two particle beams (410) oppose each other.

8. The method for producing a stent according to claim 7, **characterised in that** the particle beam (410) from the nozzle (310) inserted into the stent (100) is directed towards the edges (223) of the strut (200) facing the nozzle (310), and the nozzle (310) is withdrawn at a predetermined speed from the stent (100) so as to effect sequential irradiation of multiple struts (200) disposed side by side in the longitudinal direction for the stent.

9. The method for producing a stent according to either one of claims 7 or 8, **characterised in that** the irradiation of the edges (223) is effected bilaterally, wherein the nozzle (310) is drawn at least once from the stent (100) towards the first stent end (120) and at least once from the stent (100) towards the second stent end (130).

10. The method for producing a stent according to any one of claims 7 to 9, **characterised in that** after particle beam irradiation the stent (100) is processed by means of electropolishing.

11. The method for producing a stent according to any one of claims 8 to 10, **characterised in that** the withdrawal speed of the nozzle (310) is varied.

## Revendications

1. Stent (100) doté d'une structure de grille (110) en ressemblant à un tube comprenant des entretoises individuelles, où le stent présente au moins une entretoise (200) à partir de laquelle s'étend au moins un segment longitudinal (220) avec au moins une composante directionnelle dans la direction circonférentielle du stent et où la surface de segment longitudinal orientée vers le côté extérieur du stent est simplement recourbée autour de l'axe longitudinal (150) du stent,
**caractérisé en ce que**
le stent (100) présente simplement des entretoises (200) dans ses zones d'extrémités (122, 132) qui sont conçues de telle manière que la surface (221) du segment longitudinal d'entretoise (220) orientée vers le côté intérieur du stent présente une courbure (222) telle que la section transversale d'entretoise est optimisée fluidiquement par des bords luminaux arrondis afin d'empêcher les turbulences du flux sanguin traversant le stent,
dans lequel les zones d'extrémités s'étendent en partant d'une extrémité de stent (120, 130) respective sur respectivement 20 à 30 % de la longueur du stent (100).

2. Stent selon la revendication 1, **caractérisé en ce que** la courbure (222) de la surface de l'entretoise (200) orientée vers le côté intérieur du stent (100) est conçue asymétrique et convexe.

3. Stent selon l'une des revendications précédentes, **caractérisé en ce que** l'entretoise (200) comprend un matériau biodégradable.

4. Stent selon l'une des revendications précédentes, **caractérisé en ce que** tous les segments d'entretoises du stent (100) qui s'étendent dans la direction circonférentielle radiale du stent avec au moins une composante directionnelle sont conçus selon l'une des revendications 1 à 3.

5. Stent selon l'une des revendications précédentes, **caractérisé en ce qu'**il est construit de manière symétrique spéculaire.

6. Procédé de fabrication d'un stent (100) selon l'une des revendications 1 à 5, dans lequel la structure de grille (110) du stent est fabriquée de telle manière qu'au moins un segment longitudinal (220) d'une entretoise (200) s'étendant avec au moins une composante directionnelle dans la direction circonférentielle du stent présente une section transversale sensiblement carrée, dans lequel un arrondissement des bords (223) du segment longitudinal (220) de l'entretoise (200) luminaux et s'étendant avec au moins une composante directionnelle perpendiculaire à la direction longitudinale du stent a lieu par au moins un flux de particules orienté sur les bords (223), **caractérisé en ce que** le flux de particules sort d'une buse (310) à l'extrémité d'une conduite de transport (300) qui est insérée dans le stent (100).

7. Procédé de fabrication d'un stent selon la revendication 6, **caractérisé en ce que** les deux bords (223) luminaux du segment longitudinal d'entretoise (220) sont façonnés avec respectivement au moins un flux de particules (410), où les sens des deux flux de particules (410) sont opposés.

8. Procédé de fabrication d'un stent selon la revendication 7, **caractérisé en ce que** le flux de particules (410) sortant de la buse (310) insérée dans le stent (100) est orienté vers les bords (223) de l'entretoise (200) pointant en direction de la buse (310) et la buse (310) est retirée du stent (100) avec une vitesse déterminée pour une exposition séquentielle de plusieurs entretoises (200) disposées les unes à côté des autres dans la direction longitudinale du stent.

9. Procédé de fabrication d'un stent selon l'une des revendications 7 ou 8, **caractérisé en ce que** l'exposition des bords (223) a lieu des deux côtés, dans lequel la buse (310) est retirée au moins une fois du stent (100) en direction de la première extrémité de stent (120) et est retirée au moins une fois du stent (100) en direction de la seconde extrémité de stent (130).

10. Procédé de fabrication d'un stent selon l'une des revendications 7 à 9, **caractérisé en ce qu'**après l'exposition aux particules, le stent (100) est façonné au moyen d'un électro-polissage.

11. Procédé de fabrication d'un stent selon l'une des revendications 8 à 10, **caractérisé en ce que** la vitesse de la buse (310) varie au cours du retrait.
